## Europäisches Patentamt
## European Patent Office
## Office européen des brevets

(19)

(11) Veröffentlichungsnummer: **0 322 452**
**B1**

(12) **EUROPÄISCHE PATENTSCHRIFT**

(45) Veröffentlichungstag der Patentschrift:
13.06.90

(51) Int. Cl.⁵: **A61L 9/12**

(21) Anmeldenummer: 88907130.4

(22) Anmeldetag: 09.08.88

(86) Internationale Anmeldenummer:
PCT/CH 88/00134

(87) Internationale Veröffentlichungsnummer:
WO 89/01344 (89.02.23 Gazette 89/5)

(54) VORRICHTUNG ZUR ABDUNSTUNG VON WIRKSTOFFEN.

(30) Priorität: 31.03.88 CH 1216/88

(43) Veröffentlichungstag der Anmeldung:
05.07.89 Patentblatt 89/27

(45) Bekanntmachung des Hinweises auf die Patenterteilung:
13.06.90 Patentblatt 90/24

(84) Benannte Vertragsstaaten:
AT BE CH DE ES FR GB GR IT LI LU NL SE

(56) Entgegenhaltungen:
WO-A-80/00003
FR-A- 2 271 135
FR-A- 2 539 031

(73) Patentinhaber: Weick, Heinz Hermann, 94, rue de la Servette, CH-1202 Genf(CH)

(72) Erfinder: Weick, Heinz Hermann, 94, rue de la Servette, CH-1202 Genf(CH)

(74) Vertreter: Lauer, Joachim, Dr., Hug Interlizenz AG Austrasse 44 Postfach, CH-8045 Zürich(CH)

## Beschreibung

Die Erfindung betrifft eine Vorrichtung zur Abdunstung von Wirkstoffen. Sie betrifft insbesondere eine solche Vorrichtung mit einem plättchenförmig flachen Hohlkörper mit zwei Breitseitenwandungen, von denen wenigstens eine einen porös ausgebildeten Bereich aufweist und mit einem auf diesem gleitend verschiebbar angeordneten, schieberartigen Abdeckorgan, welches aus einer den porösen Bereich des Hohlkörpers abdeckenden Verschlussstellung in wenigstens eine, diesen Bereich zumindest teilweise freigebende Abdunstungsstellung verstellbar ist.

Eine solche Vorrichtung dient der passiven Abdunstung (z.B. von Parfum), sowie der aktiven Abdunstung (z.B. von paramedizinischen Werkstoffen gegen Erkältungsbeschwerden, zum Entwöhnen des Rauchens oder dergl.). Für eine aktive Abdunstung wird die Vorrichtung vor den Mund oder unter die Nase gehalten, um den Wirkstoff durch den porösen Abdunstbereich zu inhalieren.

Da es sich bei den Wirkstoffen meist um leicht verflüchtigende Flüssigkeiten mit teilweise recht hohem Dampfdruck handelt, zwischen der Fabrikation der Geräte und deren Erwerb durch den Endverbraucher mitunter ein grösserer Zeitraum liegt, und die Verwendungsdauer möglichst gross sein soll, ist das Verlust- bzw. Dichtigkeitsproblem von grosser Bedeutung. Eine einwandfreie Abdichtung ist auch deshalb erforderlich, weil die Gesetzgeber vieler Länder eine Produktstabilität verlangt, die je nach Land zwischen 2 - 5 Jahren betragen kann. Unter Stabilität ist im Hinblick auf Abdunstungsvorrichtungen der hier betrachteten Art insbesondere zu verstehen, dass sich die in der Vorrichtung befindliche Flüssigkeit in der gesetzlich vorgeschriebenen Zeitspanne nicht verändert, also auch nicht entweichen kann. Wenn aus irgendeinem Grunde die vorgeschriebene Zeitspanne nicht garantiert werden kann, muss das Produkt mit einem Verfalldatum versehen werden, genau wie bei Lebensmitteln.

### Stand der Technik

Es ist bisher eine Vorrichtung eingangs beschriebener Bauweise bekannt geworden. Bei dieser konnte das Problem der Gasdichtigkeit nicht zufriedenstellend gelöst werden. Insbesondere schliesst das Abdeckorgan in seiner Verschlussstellung nicht genügend gasdicht ab. Man hat daher den Abdichtbereich zwischen dem Hohlkörper und dem Abdeckorgan mit einem selbstklebenden Dichtungsband aus Aluminiumfolie umgeben, die verhältnismässig teuer und auf Grund der flachen Geräteform nur schwer zu plazieren ist. Die dadurch erzielte Verbesserung der Dichtigkeit erwies sich jedoch als ausgesprochen ungenügend. Eine ausreichend gasdichte Verklebung ist mit Selbstklebeband nicht erreichbar. Zum Ausgleich der Oberflächenrauhigkeit von Hohlkörper und Abdeckorgan muss viel Kleber verwendet werden, was nach dem Entfernen des Dichtungsbandes auf der Vorrichtung unerwünschte Rückstände zur Folge hat. Die durch das Dichtungsband erzielte Verbesserung der Dichtigkeit ist weiter nur bis zur erstmaligen Geräteverwendung durch den Verbraucher gegeben. Der anschliessend wieder auftretende höhere Verlust verkürzt nach wie vor erheblich die Zeitspanne der Benützung. Der hohe Preis der Wirkstoffe macht diesen Zustand noch unbefriedigender: dem Kunden steht nur ein Teil von dem von ihm bezahlten Inhalt zur Verfügung.

### Darstellung der Erfindung

Der Erfindung liegt insbesondere die Aufgabe zugrunde, bei einer Vorrichtung der eingangs genannten Art, die Dichtigkeit mit möglichst einfachen Mitteln zu erhöhen. Diese sowie weitere Aufgaben werden gemäss der vorliegenden Erfindung gelöst durch eine Vorrichtung mit den Merkmalen des Patentanspruchs 1.

Erfindungsgemäss ist demnach der Hohlkörper mit einer umlaufenden, zum Abdeckorgan hin offenen, sich nach aussen erweiternden Einschubdichtungsnute und das Abdeckorgan zum Zwecke seines Eingriffs mit einem Randabschnitt in diese Einschubdichtungsnute in seiner Verschlussstellung an diesem Randabschnitt aussenseitig mit einer zur Einschubdichtungsnute gegengleichen Verjüngung versehen. Weiter sind zumindest die äussere Oberfläche des Hohlkörpers im Bereich der Einschubdichtungsnute und zumindest die innere Oberfläche des Abdeckorgans im Bereich seines Randabschnitts als glatte, in der Verschlussstellung dicht aneinander anliegende Dichtflächen ausgebildet. Schliesslich ist der Hohlkörper im Bereich der Einschubdichtungsnute und seiner darin angeordneten Dichtfläche ringsherum geringfügig verstärkt, so dass die genannte Dichtfläche zumindest gegenüber dem porösen Bereich des Hohlkörpers etwas erhaben ist.

Wird das auf dem Hohlkörper gleitende Abdeckorgan in seine Verschlussposition geschoben, so gelangt dessen verjüngter Randabschnitt in die Einschubdichtungsnute. Da sich diese nach innen zu verjüngt, kann der Randabschnitt ab einer gewissen Einschubstellung nur noch unter elastischer Verformung seiner selbst sowie hauptsächlich der Einschubdichtungsnute weiter in diese hineingeschoben werden. Dabei werden die genannten Dichtflächen am Hohlkörper sowie am Abdeckorgan elastisch aufeinandergepresst, wodurch sich eine ausgezeichnete Dichtwirkung ergibt.

Beim Einschieben des Abdeckorgans mit seinem Randbereich in die Einschubdichtungsnute fährt dieser Randbereich auf die geringfügig erhöhte Dichtfläche auf. Die Erhöhung der Dichtfläche bewirkt in vorteilhafter Weise eine gewisse Erhöhung der elastischen Spannung, mit der die Dichtflächen am Hohlkörper im Bereich der Einschubdichtungsnute sowie am Abdeckorgan an dessen Randabschnitt in der Verschlussstellung aufeinander gepresst werden und garantiert darüberhinaus, dass diese Flächen überall einwandfrei aneinander anliegen. Weiter wird durch diese Erhöhung erreicht, dass das Abdeckorgan über dem übrigen, insbesondere dem porös ausgebildeten Bereich des Hohlkörpers leichtgängig verschieblich ausgebildet sein kann.

Eine bevorzugte Ausgestaltung der Erfindung liegt darin, dass wenigstens die porös ausgebildete Breitseitenwandung des Hohlkörpers und die auf dieser gleitende Wandung des Abdeckorgans durch winzige Abstandsgleitrippen auf Abstand gehalten sind, welche eine geringere Höhe aufweisen, als die Dichtfläche am Hohlkörper zumindest gegenüber dessen porösen Bereich erhaben ist. Diese Abstandsgleitrippen verhindern, dass ein z.B. auf dem Wege der Kondensierung zwischen Abdeckorgan und Breitseitenwandung gelangendes Wirkstofftröpfchen infolge der Verschiebung des Abdeckorgans zu einer flächigen Benetzung führen kann.

Gemäss einer weiteren bevorzugten Ausgestaltung der Erfindung ist der Hohlkörper aus einem ersten und einem zweiten Teil zusammengesetzt, wobei der zweite Teil den ersten Teil mit einem Endabschnitt umgreift, und wobei die Einschubdichtungsnute durch eine innenseitige Verjüngung am genannten Endabschnitt des zweiten Teils des Hohlkörpers gebildet wird. Bei einer solchen zweiteiligen Ausführung des Hohlkörpers sind vorzugsweise dessen erster und zweiter Teil in ihrem Überlappungsbereich mittels einer umlaufenden Schweissnaht miteinander verschweisst. Zur Herstellung der Schweissnaht ist dabei weiter vorzugsweise der erste Teil des Hohlkörpers umfangsseitig mit einer umlaufenden dünnen Schweissrippe versehen, die in einen etagenförmigen Absatz im Endabschnitt des zweiten Teils des Hohlkörpers eingreift. Die Schweissnaht sollte um nicht mehr als die Tiefe der Einschubdichtungsnute von dieser entfernt angeordnet ist. Insbesondere durch eine solche Lage der Schweissnaht ergibt sich im Bereich der Einschubdichtungsnute eine erhebliche Versteifung, welche sich durch Erhöhung der vorgenannten elastischen Spannungen in der Verschlussstellung des Abdeckorgans weiter verstärkend auf die Dichtigkeit auswirkt.

Die vorgenannten sowie weitere vorteilhafte Ausgestaltungen und Weiterbildungen der vorliegenden Erfindung sind in den abhängigen Ansprüchen gekennzeichnet.

## Kurze Beschreibung der Zeichnungen

In der Zeichnung ist ein Ausführungsbeispiel der Erfindung dargestellt, aus dessen Beschreibung die durch die Erfindung erzielten Vorteile sowie auch die vorstehend erwähnten bevorzugten Ausgestaltungen und Weiterbildungen klar hervorgehen. Es zeigen:

Fig. 1 einen teilgeschnittenen Seitenriss der Vorrichtung,
Fig. 2 einen Schnitt A-A nach Fig. 1,
Fig. 3 einen Schnitt B-B nach Fig. 1,
Fig. 4 einen Schnitt C-C nach Fig. 1 und
Fig. 5 einen Schnitt D-D nach Fig. 1.

In den einzelnen Figuren sind übereinstimmende Teile mit gleichen Bezugszeichen versehen.

Zur Erzielung einer grösstmöglichen Klarheit der einzelnen Konstruktionsdetails wurden die Figuren in verschiedenen Massstäben dargestellt. Demgegenüber ist die Vorrichtung in der Ausbildung zum Tragen in Taschen von Kleidungsstücken so bemessen, dass sie z.B. auch in einer kleinen Brusttasche eines Hemdes oder einer Bluse Platz findet. Für Sockelgeräte ist die Grösse von weniger grosser Bedeutung.

## Bester Weg zur Ausführung der Erfindung

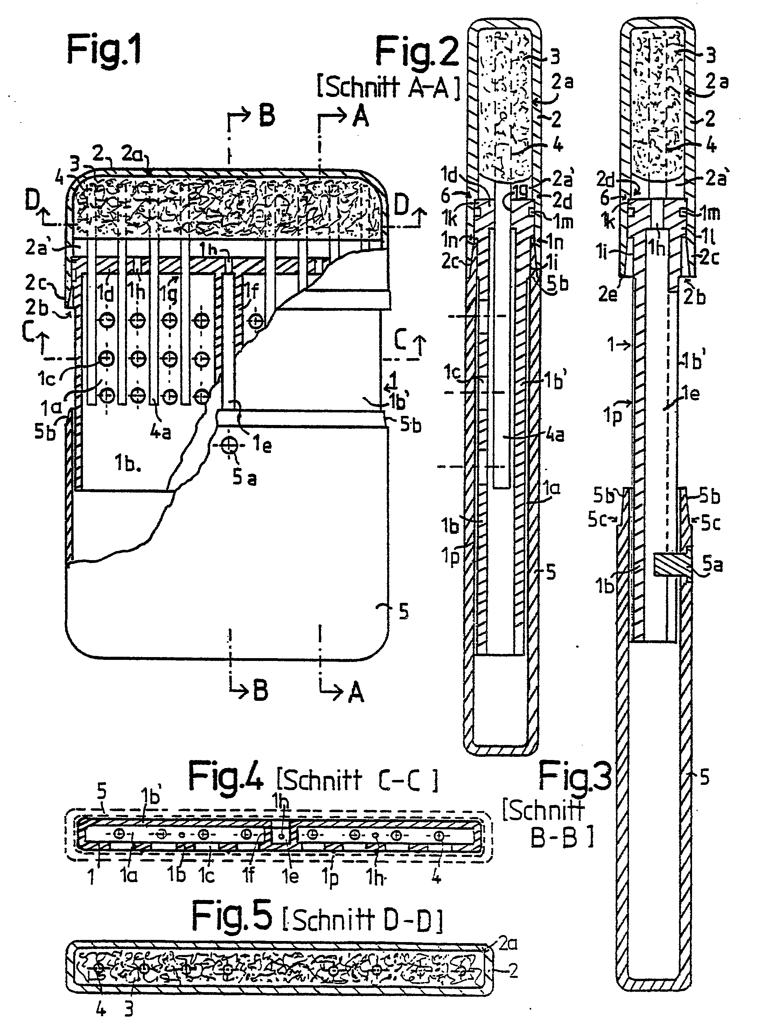

Es wird nunmehr auf die Zeichnungen Bezug genommen. Der darin dargestellte plättchenförmig flache Hohlkörper 1,2 besteht aus dem eine Abdunstkammer 1a umschliessenden ersten Teil 1 (Abdunstkammerteil) und dem zugleich als Griffteil dienenden, eine Wirkstoffreservekammer 2a enthaltenden zweiten Teil 2 (Reservekammerteil). Diese beiden Teile sind durch eine umlaufende Ultraschallverschweissung gegeneinander befestigt. Die Reservekammer 2a ist bis auf den schmalen Freiraum 2a' von einem hülsenförmig umschlossenen Tampon 3 aus saugfähigem Material ausgefüllt. Abdunstkammer 1a und Reservekammer 2a sind durch eine Querwand 1d voneinander getrennt, die an das in den zweiten Teil 2 hineinragende Ende des ersten Teiles 1, angeformt ist. In der Querwand 1d sind Durchtrittskanäle 1g vorgesehen. Durch diese führen dochtartige Wirkstoffleiter 4. Die dochtartigen Wirkstoffleiter 4 ragen mit ihren in den Figuren 1 - 3 oberen Längsbereichen in den Tampon 3. Mit ihren in den genannten Figuren unteren Bereichen 4a erstrecken sie sich in die Abdunstkammer 1a hinein und über nehmen dort unmittelbar die Abdunstfunktion. Sie sind von im wesentlichen formstabiler, gradliniger Ausbildung und bestehen vorzugsweise aus Chemiefasern.

In der Querwand 1d befinden sich noch Druckausgleichsöffnungen 1h, die zudem zum Auffüllen der Reservekammer 2a mittels Einspritzkanülen dienen. Die eine 1b der beiden Breitseitenwandungen 1b, 1b' des Abdunstkammerteiles 1 ist mit in Reihen angeordneten Abdunstlöchern 1c versehen und dadurch porös ausgebildet. Das Abdeckorgan 5 ist als schieberartiger, den Abdunstkammerteil 1 des Hohlkörpers 1,2 eng umschliessender, auf diesem gleitender Körper ausgebildet. Er besitzt zur Begrenzung seiner maximalen Auszugsposition innenseitig einen mittig angeordneten zylindrischen Anschlagnocken 5a, der in eine Anschlaglängsnute 1e des Abdunstkammerteils 1 eingreift. Diese Nute wird beidseitig durch Stege 1f begrenzt und weist keine Verbindung zur Abdunstkammer 1a auf.

Die Längsbereiche 4a der Wirkstoffleiter 4 weisen einen gewissen Abstand von den Seitenwandungen 1b, 1b' des Abdunstkammerteiles 1 auf. Dadurch ist eine unerwünschte Benetzung der genannten Wandungen verhindert. Damit die Wirkstoffleiter 4 in dieser Lage verharren, sind sie, wie schon erwähnt, im wesentlich formstabil und gradling ausgebildet und jeweils geschützt zwischen den Längsreihen der Abdunstlöcher 1c angeordnet. Zudem sichern die Stege 1f des Abdunstkammerteiles 1 den gegenseitigen Abstand der Wandungen 1b, 1b'. Nicht unbedingt erforderlich ist allerdings, dass die Wirkstoffleiter absolut keine Berührung mit den Seitenwandungen aufweisen. Bei einem bevorzugten Abstand von nur einigen 1/10 mm ist eine gewisse gegenseitige Berührung, beispielsweise schon infolge geringfügiger Durchbiegung der Wirkstoffleiter, nicht immer ganz zu vermeiden. Eine solche Berührung bleibt jedoch zumindest dann ohne nachteilige Folgen und ist tolerierbar, wenn sie mehr oder weniger nur punktuell ist.

Der Uebergangsbereich zwischen dem Abdunstkammerteil 1 und dem Reservekammerteil 2, sowie die zwischen Hohlkörper 1,2 und Abdeckorgan 5 in dessen Verschlussposition dichtend zusammenwirkenden Bereiche besitzen eine ganz spezielle, neuartige Ausbildung. So weist zunächst der Reservekammerteil 2 im Bereich seines den Abdunstkammerteil 1 umgreifenden Endabschnitts zur Bildung einer umlaufenden, zum Abdeckorgan 5 hin offenen, sich nach aussen erweiternden Einschubdichtungsnute 2b die innenseitige, konusartige Verjüngung 2c auf. Zum Zwecke des Eingriffs in diese ist der Randabschnitt bzw. Oeffnungsbereich des Abdeckorgans 5 aussenseitig mit der gegengleichen konusartigen Verjüngung 5b versehen.

Die äussere Oberfläche des Abdunstkammerteils ist im Bereich der Einschubdichtungsnute und die innere Oberfläche des Abdeckorgans zumindest im Bereich seines Randabschnitts als glatte Dichtfläche ausgebildet. In der Verschlussstellung (Fig. 2) liegen die genannten Dichtflächen dicht aneinander an. Die Rauhtiefe der genannten Dichtflächen beträgt zwischen etwa 2/1000 mm und etwa 3/1000 mm. Des weiteren ist die Wandstärke des Abdunstkammerteils 1 im Bereich 1i der Einschub dichtungsnute 2b und damit der Dichtfläche ringsherum geringfügig, vorzugsweise um einige wenige 1/10 mm, verstärkt.

Auch die konusartig verjüngten Flächen 2c und 5b sind als entsprechend glatte Dichtflächen ausgebildet.

An den verjüngten Randbereich 5b des Abdeckorgans 5 schliesst sich ein nach aussen springender Absatz 5c an, welcher in der Verschlussstellung des Abdeckorgans, wenn dessen genannter Randbereich in die Einschubdichtungsnute 2b eingreift, am Oeffnungsrand 2e der Einschubdichtungsnute anliegt. Der Absatz 5c ist so angeordnet, dass das Abdeckorgan mit seinem Randabschnitt zum Erreichen der Verschlussstellung etwas in die Einschubdichtungsnute unter gewisser elastischer Verformung derselben hineingedrückt werden muss. Die Tiefe der Einschubdichtungsnute 2b ist derart bemessen, dass in dieser Stellung in ihrem hinteren Bereich noch ein kleiner umlaufender Hohlraum 1n verbleibt.

Die umlaufende Ultraschallverschweissung ist mit 6 bezeichnet. Sie ist um nicht mehr als die Tiefe der Einschubdichtungsnute von dieser entfernt angeordnet. Dadurch ergibt sich eine gewünscht hohe Steifigkeit des Reservekammerteils 2 im Bereich der Einschubdichtungsnute 2b. Zur Herstellung der Schweissnaht ist an den Abdunstkammerteil 1 umfangsseitig ringsherum eine umlaufende dünne Schweissrippe 1k angeformt.

Diese liegt an dem etagenförmigen Absatz 2d des Reservekammerteiles 2 an. Diese speziellen schweisstechnischen Konstruktionsmassnahmen erlauben eine perfekte gasdichte Verschweissung, obwohl diese zur Vermeidung von Verformungen des sehr dünnwandigen und durch die Abdunstlöcher 1c geschwächten Abdunstkammerteils 1 mit sehr geringer Schweissenergie durchgeführt werden muss. Infolge der Ausrichtung und der geringen Dicke der Schweissrippe 1k wird im Schweisskontaktbereich ein optimales Schwingungspotential wirksam, wodurch sich die Schweissrippe 1k federnd gegen den Absatz 2d drückt und sich bei gleichzeitiger Verformung gasdicht mit diesem verbindet.

Zwischen der Verschweissung 6 und der Einschubdichtungsnute 2b befindet sich noch eine schmale, durch die Anformung der Schweissrippe 1k und einer weiteren Rippe 1l bedingte umlaufende Nute 1m.

Die erzielten Dichtungsverhältnisse sind folgende:
Wird das leicht auf dem Abdunstkammerteil 1 gleitende Abdeckorgang 5 in seine Verschlussposition geschoben, so gelangt dessen verjüngter Randabschnitt bzw. Oeffnungsbereich 5b in die Einschubdichtungsnute 2b, während er gleichzeitig auf den verstärkten Bereich 1i des Abdunstkammerteils 1 auffährt. Da die Wandungen des Reservekammerteiles 2 aufgrund der Verschweissung 6 und ihrer Lage nur geringfügig, d.h. nur im gewollten Masse nachgeben, liegt der Randabschnitt des Abdeckorgans 5 fest in der Einschubdichtungsnute 2b eingeklemmt, in der noch ein kleiner Hohlraum 1n verbleibt. Als Anschlag für das Abdeckorgan 5 dient der Oeffnungsrand 2e des Reservekammerteils 2.

Vom Abdunstraum 1a aus bestehen somit für das sich in diesem befindende abgedunstete Wirkstoffgas zwei hintereinander geschaltete Dichtflächen: eine innere Dichtfläche zwischen dem verstärkten Wandungsbereich 1i und der Innenfläche des Randabschnitts des Abdeckorgans 5 sowie eine äussere Dichtfläche zwischen der Verjüngung 5b und der Verjüngung 2c. Im Vergleich miteinander weist die innere Dichtfläche eine in der Regel deutlich bessere und auch sicherere Dichtwirkung als die äussere Dichtfläche auf. Falls in den Bereich der inneren Dichtfläche Kondensflüssigkeit gelangt, so kann diese infolge Kapillarwirkung nur bis zu dem die genannte Wirkung unterbrechenden Hohlraum 1n vordringen. Die Dichtung ist also selbst in Bezug auf eine Benetzung des inneren Dichtungsbereichs mit kondensiertem Wirkstoff praktisch perfekt.

Durch die gasdichte Verschweissung 6 ist von der Reservekammer 2a her ein Verlust von flüssigem oder auch bereits vergastem Wirkstoff an sich ausgeschlossen. Sollte jedoch einmal eine Verschweissung nicht perfekt sein, so ist die erforderliche Dichtigkeit trotzdem durch die die Kapillarwirkung unterbrechende Nute 1m und die mit diesen in Reihe liegende äussere Dichtfläche zwischen den beiden Verjüngungen 2c, 5b in ausreichendem Masse gegeben.

Insbesondere aus den Figuren 2 bis 4 geht noch hervor, dass aussen auf der Breitseitenwandung 1b zwischen den Längsreihen der Abdunstlöcher 1c

noch winzige Abstandsgleitrippen 1p vorgesehen sind. Diese verhindern, dass ein z.B. auf dem Wege der Kondensierung zwischen Abdeckorgan 5 und die Seitenwandung 1b gelangendes Wirkstofftröpfchen infolge der Verschiebung des Abdeckorgans 5 zu einer flächigen Benetzung führen kann. Obwohl eine solche Benetzung in erster Linie auf der Seite der Abdunstlöcher auftreten kann, lassen sich entsprechende Rippen natürlich auch aussenseitig der Seitenwandung 1b' anformen. Alternativ können sich solche Rippen auf den Innenflächen des Abdeckorgans 5 befinden. Wichtig ist, dass die Abstandsgleitrippen eine geringere Höhe (etwa 1/10 - 2/10 mm) aufweisen als die Dichtfläche im verstärkten Wandungsbereich 1i am Hohlkörper zumindest gegenüber dessen porösen Bereich erhaben ist, damit die Wirkung dieser Verstärkung erhalten bleibt.

Bei der beschriebenen Vorrichtung ist nur die Breitseitenwandung 1b des Abdunstkammerteiles 1 mit Abdunstlöchern 1c versehen. So ausgebildet, dient das Gerät bevorzugt als Mund- und Naseninhalator (z.B. zur Abdunstung eines die Atemwege erfrischenden, bzw. eines heilwirksamen Wirkstoffes wie Eukalyptusöl bei entzündlichen Erkrankungen der Atemwege, eines Wirkstoffes zum Entwöhnen des Rauchens oder dergl.). Zum Inhalieren wird die Vorrichtung mit ihrer die Abdunstlöcher 1c enthaltenden Seite unmittelbar gegen den Mund oder unter die Nase gehalten. Während des Einatmens dringt Aussenluft durch die nicht von Mund oder Nase abgedeckten Abdunstlöcher in den Abdunstraum 1a, streicht parallel zu den Breitseitenwandungen 1b, 1b' an den Wirkstoffleiterbereichen 4a vorbei und gelangt - intensiv mit Wirkstoff angereichert - in den entsprechenden Atemweg.

Soll die Vorrichtung zum Abdunsten von Parfum dienen, so ist die Abdunstwirkung verständlicherweise am grössten, wenn beide Breitseitenwandung 1b, 1b' mit Abdunstlöchern 1c versehen sind, da bei dieser Verwendung die Luft nicht zwangsweise die Abdunstkammer 1a durchströmt. Als Parfum-Abdunster eignet sich die Vorrichtung mittels eines separaten oder angeformten Sockels (die Reervekammer könnten sich im Sockelbereich befinden) oder mittels eines Stützfusses zum Plazieren auf dem Schreibtisch oder dergl.. Als Sockelgerät kann die Vorrichtung natürlich grösser bemessen sein, als wenn sie zum Mitführen in Taschen von Kleidungsstücken bestimmt ist.

**Patentansprüche**

1. Vorrichtung zur Abdunstung von Wirkstoffen mit einem plättchenförmig flachen Hohlkörper mit zwei Breitseitenwandungen, von denen wenigstens eine einen porös ausgebildeten Bereich aufweist und mit einem auf diesem gleitend verschiebbar angeordneten, schieberartigen Abdeckorgan, welches aus einer den porösen Bereich des Hohlkörpers abdeckenden Verschlussstellung in wenigstens eine, diesen Bereich zumindest teilweise freigebende Abdunststellung verstellbar ist, dadurch gekennzeichnet, dass der Hohlkörper (1, 2) mit einer umlaufenden, zum Abdeckorgan (5) hin offenen, sich nach aussen erweiternden Einschubdichtungsnute (2b) versehen ist, dass das Abdeckorgan (5) zum Zwecke seines Eingriffs mit einem Randabschnitt in diese Einschubdichtungsnute (Verschlussstellung) an diesem Randabschnitt aussenseitig eine zur Einschubdichtungsnute (2b) gegengleiche Oberfläche des Hohlkörpers (1, 2) im Bereich der Einschubdichtungsnute und zumindest die innere Oberfläche des Abdeckorgans im Bereich seines Randabschnittes als glatte, in der Verschlussstellung dicht aneinander anliegende Dichtflächen ausgebildet sind und dass der Hohlkörper (1, 2) im Bereich der Einschubdichtungsnute (2b) und seiner darin angeordneten Dichtfläche ringsherum geringfügig verstärkt ist, so dass die genannte Dichtfläche zumindest gegenüber dem porösen Bereich des Hohlkörpers (1, 2) etwas erhaben ist.

2. Vorrichtung nach Anspruch 1, dadurch gekennzeichnet, dass wenigstens die porös ausgebildete Breitseitenwandung (1b) des Hohlkörpers (1, 2) und die auf dieser gleitende Wandung des Abdeckorgans (5) durch winzige Abstandsgleitrippen (1p) auf Abstand gehalten sind.

3. Vorrichtung nach Anspruch 1 und 2, dadurch gekennzeichnet, dass die Abstandsgleitrippen (1p) eine geringere Höhe aufweisen als die Dichtfläche am Hohlkörper (1, 2) zumindest gegenüber dessen porösen Bereich erhaben ist.

4. Vorrichtung nach einem der Ansprüche 1 bis 3, dadurch gekennzeichnet, dass sich an den verjüngten Randbereich des Abdeckorgans (5) ein nach aussen springender Absatz (5c) anschliesst, welcher in der Verschlussstellung des Abdeckorgans (5) am Öffnungsrand (2e) der Einschubdichtungsnute (2b) anliegt, wobei der genannte Randbereich in die Einschubdichtungsnute elastisch eingreift und dass die Tiefe der Einschubdichtungsnute (2b) derart bemessen ist, dass in dieser Stellung in ihrem hinteren Bereich ein kleiner umlaufender Hohlraum (1n) verbleibt.

5. Vorrichtung nach einem der Ansprüche 1 bis 4, dadurch gekennzeichnet, dass der Hohlkörper (1, 2) aus einem ersten (1) und einem zweiten Teil (2) zusammengesetzt ist, wobei der zweite Teil (2) den ersten Teil (1) mit einem Endabschnitt umgreift und wobei die Einschubdichtungsnute (2b) durch eine innenseitige Verjüngung (2c) am genannten Endabschnitt des zweiten Teils (2) des Hohlkörpers (1, 2) gebildet wird.

6. Vorrichtung nach Anspruch 5, dadurch gekennzeichnet, dass der erste (1) und der zweite Teil (2) des Hohlkörpers (1, 2) in ihrem Überlappungsbereich mittels einer umlaufenden Schweissnaht (6) miteinander verschweisst sind, dass zur Herstellung der Schweissnaht (6) der erste Teil (1) des Hohlkörpers (1, 2) umfangsseitig mit einer umlaufenden dünnen Schweissrippe (1k) versehen ist, die in einen etagenförmigen Absatz (2d) im Endabschnitt des zweiten Teils (2) des Hohlkörpers (1, 2) eingreift.

7. Vorrichtung nach Anspruch 6, dadurch gekennzeichnet, dass die Schweissnaht (6) um nicht mehr als die Tiefe der Einschubdichtungsnut (2b) von dieser entfernt angeordnet ist.

8. Vorrichtung nach einem der Ansprüche 5 bis 7, dadurch gekennzeichnet, dass der erste Teil (1) des Hohlkörpers (1, 2) im Überlappungsbereich mit dem zweiten Teil (2) des Hohlkörpers (1, 2) mit wenigstens einer umlaufenden Sperrnute (1m) versehen ist.

9. Vorrichtung nach einem der Ansprüche 6 bis 8, dadurch gekennzeichnet, dass der Innenraum des Hohlkörpers (1, 2) durch eine Querwand (1d) in eine vom seinem ersten Teil umschlossenen Abdunstkammer (1a) und eine von seinem zweiten Teil umschlossene Reservekammer (2a) unterteil ist und dass die Querwand (1d) an das in seinen zweiten Teil (2) hineinragende Ende seines ersten Teiles (1), unmittelbar reservekammerseitig jenseits der umlaufenden Schweissnaht (6) angeformt ist.

10. Vorrichtung nach Anspruch 9, dadurch gekennzeichnet, dass die Querwand (1d) mit mindestens einem Durchtrittskanal (1h) versehen ist, dass sich durch diesen Durchtrittskanal (1h) ein mit seinem Ende in der Reservekammer (2a) eingebetteter Wirkstoffleiter (4) in die Abdunstkammer (1a) hinein erstreckt, dass der Wirkstoffleiter (4) von im wesentlichen formstabiler, gradliniger Ausbildung und bezüglich seines Durchmessers derart bemessen ist, dass er sich in der Abdunstkammer (1a) weitgehend berührungsfrei von deren Breitseitenwandungen (1b, 1b') erstreckt und dass die Reservekammer (2a) mit einem saugfähigen Material (3) gefüllt ist.

**Claims**

1. A device for the atomizing of active substances, with a plate-form flat hollow body with two wide-side walls, of which at least one has a zone designed porous, and with a slide-type cover element, arranged to move with sliding on same, which is adjustable from a closed position, covering the porous zone of the hollow body, to at least one atomizing position, at least partly freeing this zone, with the distinction that the hollow body (1, 2) is provided with a push-in sealing groove (2b), circumferential, open toward the cover element (5) and widening toward the outside, that the cover element (5) has, for the purpose of its engagement with a rim section in this push-in sealing groove (closed position), on its outside, at this rim section, a tapering (5b) corresponding to the push-in sealing groove (2b) that at least the outer surface of the hollow body (1, 2), in the zone of the push-in sealing groove, and at least the inner surface of the cover element in the zone of its rim section, are designed as smooth sealing surfaces, lying tightly against each other in the closed position and with the distinction that the hollow body (1, 2) in the zone of the push-in sealing groove (2b) and its sealing surface arranged therein is slightly reinforce, circumferentially so that the said sealing surface is at least somewhat raised in relation to the porous zone of the hollow body (1, 2).

2. The device according to claim 1, with the distinction that at least the wide-side wall (1b), designed porous, of the hollow body (1, 2) and the wall of the cover element (5), sliding on the latter, are held at a distance by tiny distanced slide ridges (1p).

3. The device according to claim 1 and 2, with the distinction that the distanced sliding ridges (1p) are less high than the sealing surfaces on the hollow body (1, 2), is raised at least opposite its porous zone.

4. The device according to one of claims 1 to 3, with the distinction that to the tapered rim zone of the cover element (5) adjoins an offset (5c), springing outward, which in the closed position of the cover element (5) lies against the rim (2e) of the opening of the push-in sealing groove (2b), while the said rim zone engages elastically in the push-in sealing groove, and that the depth of the push-in sealing grove (2b) is so dimensioned that in this position, there remains in its rear zone a small circumferential hollow space (1n).

5. The device according to one of claims 1 to 4, with the distinction that the hollow body (1, 2) is composed of a first (1) and a second part (2), the second part (2) gripping around the first part (1) by an end section, and the push-in sealing groove (2b) being formed by an inside tapering (2c) on the said end section of the second part (2) of the hollow body (1, 2).

6. The device according to claim 5, with the distinction that the first (1) and the second parat (2) of the hollow body (1, 2) are welded together in their overlapping zone, by means of a circumferential welding seam (6), that for the production of the welding seam (6), the first part (1) of the hollow body (1, 2) is provided peripherally with a circumferential thin ridge of welding (1k), which engages in a step-form (graduated) offset (2d) in the end section of the second part (2) of the hollow body (1, 2).

7. The device according to claim 6, with the distinction that the welding seam (6) is distanced from the push-in sealing groove (2b) by not more than the depth of the latter.

8. The device according to one of claims 5 to 7, with the distinction that the first part (1) of the hollow body (1, 2), in the overlapping zone with the second part (2) of the hollow body (1, 2), is provided with at least one circumferential blocking groove (1m).

9. The device according to one of claims 6 to 8, with the distinction that the inner space of the hollow body (1, 2) is divided by a transverse wall (1d) into an atomization chamber (1a), enclosing its first part (1) and a reserve chamber (2a), enclosing its second part, and that the transverse wall (1d) is formed, on the end of its first part (1), projecting into its second part (2), directly opposite of the circumferential welding seam (6) on the reserve chamber side.

10. The device according to claim 9, with the distinction that the transverse wall (1d) is provided with at least one passagae channel (1h), that through this passage channel (1h) extends into the atomizing chamber (1a), a conductor (4) of active substance, embedded by one end in the reserve chamber (2a), that the active substance conductor (4) is of essentially form-stable, straight-line design, and is so dimensioned in its diameter that it extends into the atomizing chamber (1a) largely free of contact with the wide-side walls of the latter (1b, 1b'), and that the reserve chamber (2a) is filled with an absorptive material (3).

## Revendications

1. Dispositif de nébulisation de substances actives, comportant un corps creux, plat en forme de plaquette, comportant deux parois de grandes dimensions, dont une au moins présente une zone poreuse, et comportant un organe de recouvrement en forme de tiroir, placé coulissant sur cette zone, lequel organe se déplace d'une position de fermeture, recouvrant la zone poreuse du corps creux, dans au moins une position de nébulisation, libérant au moins partiellement cette zone, caractérisé en ce que le corps creux (1, 2) est pourvu d'une rainure d'étanchéité par insertion (2b) continue, ouverte vers l'organe de recouvrement (5), s'élargissant vers l'extérieur, en ce que l'organe de recouvrement (5) présente, à l'extérieur, sur une portion de bordure, un rétrécissement (5b) correspondant à l'opposé, à la rainure d'étanchéité par insertion (2b), pour venir en prise, par cette portion de bordure, avec la rainure d'étanchéité par insertion (position de fermeture) en ce qu'au moins la surface extérieure du corps creux (1, 2), dans la région de la rainure d'étanchéité par insertion, et au moins la surface intérieure de l'organe de recouvrement, dans la région de sa portion de bordure, sont des surfaces d'étanchéité lisses, s'appliquant étroitement l'une contre l'autre, en position de fermeture et que le corps creux (1, 2) est légèrement renforcé tout autour, dans la zone de la rainure d'étanchéité par insertion (2b) et de sa surface d'étanchéité placée à l'intérieur, de sorte que la surface d'étanchéité est légèrement en relief, au moins par rapport à la zone poreuse du corps creux (1, 2).

2. Dispositif selon la revendication 1, caractérisé en ce qu'au moins la paroi de grandes dimensions (1b) poreuse du corps creux (1, 2) et la paroi de l'organe de recouvrement (5), coulissant sur celle-ci, sont maintenues écartées, par de petites glissières d'écartement (1p).

3. Dispositif selon les revendications 1 et 2, caractérisé en ce que les glissières d'écartement (1p) ont une hauteur qui est inférieure à la distance sur laquelle la surface d'étanchéité, contre le corps creux (1, 2), est en relief, au moins par rapport à sa zone poreuse.

4. Dispositif selon l'une quelconque des revendications 1 à 3, caractérisé en ce qu'à la zone de bordure de l'organe de recouvrement (5), fait suite un appendice (5c), saillant vers l'extérieur, qui en position de fermeture de l'organe de recouvrement (5), s'applique contre le bord d'ouverture (2e) de la rainure d'étanchéité par insertion (2b), cette zone de bordure s'engageant de manière élastique, dans la rainure d'étanchéité par insertion, et en ce que la profondeur de la rainure d'étanchéité par insertion (2b) est telle que, dans cette position, il reste une petite cavité (1n) continue, dans sa zone arrière.

5. Dispositif selon l'une quelconque des revendications 1 à 4, caractérisé en ce que le corps creux est constitué d'une première partie (1) et d'une deuxième partie (2), la deuxième partie (2) entourant la première partie (1), par une portion terminale et la rainure d'étanchéité par insertion (2b) étant formée par un rétrécissement (2c) intérieur, sur la portion terminale de la deuxième partie (2) du corps creux (1, 2).

6. Dispositif selon la revendication 5, caractérisé en ce que la première partie (1) et la deuxièMe partie (2) du corps creux (1, 2) sont soudées entre elles, dans leur zone de recouvrement, au moyen d'un cordon de soudure (6) continu, en ce que pour réaliser le cordon de soudure (6), la première partie (1) du corps creux (1, 2) est pourvue, sur son pourtour, d'une mince nervure de soudage (1k) continue, qui s'engage dans un appendice (2d) étagé, dans la portion de bordure de la deuxième partie (2) du corps creux (1, 2).

7. Dispositif selon la revendication 6, caractérisé en ce que le cordon de soudure (6) est éloigné de la rainure d'étanchéité par insertion (2b), d'une distance qui n'est par supérieure à la profondeur de cette rainure.

8. Dispositif selon l'une quelconque des revendications 5 à 7, caractérisé en ce que la première partie (1) du corps creux (1, 2) est pourvue, dans la zone de recouvrement avec la deuxième partie (2) du corps creux (1, 2), d'au moins une rainure d'arrêt (1m) continue.

9. Dispositif selon l'une quelconque des revendications 6 à 8, caractérisé en ce que l'intérieur du corps creux (1, 2) est partagé par une cloison transversale (1d), en une chambre de nébulisation (1a), enfermée par sa première partie, et en une chambre de réserve (2a), enfermée par sa deuxième partie, et en ce que la cloison transversale (1d) est formée à l'extrémité de sa première partie (1), pénétrant dans sa deuxième partie (2), directement du côté de la chambre de réserve, de l'autre côté du cordon de soudure (6) continu.

10. Dispositif selon la revendication 9, caractérisé en ce que la cloison transversale (1d) est pourvue d'au moins un canal de passage (1h), en ce qu'un déflecteur de substances actives (4), encastré par l'une de ses extrémités dans la chambre de réserve (2a), s'étend, à travers ce canal de passage (1h), dans la chambre de nébulisation (1a), en ce que le déflecteur de substances actives (4) est essentiellement indéformable, rectiligne et présente un diamètre tel qu'il s'étend dans la chambre de nébulisation (1a), pratiquement sans contact avec les parois de grandes dimensions (1b, 1b'), et en ce que la chambre de réserve (2a) est remplie d'un matériau (3) absorbant.

EP 0 322 452 B1

Fig.1

Fig.2
[Schnitt A-A]

Fig.3
[Schnitt B-B]

Fig.4 [Schnitt C-C]

Fig.5 [Schnitt D-D]